# EUROPEAN PATENT SPECIFICATION

(11) **EP 3 498 330 B1**
(45) Date of publication and mention of the grant of the patent: **12.03.2025**
(21) Application number: 17888529.9
(22) Date of filing: 29.12.2017
(51) Int. Cl.: A61M 37/00, A61K 9/00

(54) **MICRONEEDLE ARRAY**
MIKRONADELARRAY
RÉSEAU DE MICRO-AIGUILLES

(30) Priority: 29.12.2016 KR 20160182699; 28.12.2017 KR 20170182712
(43) Date of publication of application: 19.06.2019
(73) Proprietor: Labnpeople Co.,Ltd., Yangju-si, Gyeonggi-do 11477 (KR)
(72) Inventor: CHO, Sung Youn, Uijeongbu-si Gyeonggi-do 11786 (KR)
(74) Representative: Simmons & Simmons LLP (Munich)
(86) International application number: PCT/KR2017/015724
(87) International publication number: WO 2018/124808

(56) References cited:
- EP-A1- 2 090 331
- WO-A1-2016/168847
- KR-A- 20060 029 162
- KR-B1- 100 682 534
- US-A1- 2002 177 858
- US-A1- 2007 049 901
- US-A1- 2007 161 964
- US-A1- 2007 161 964
- US-A1- 2007 293 815
- US-A1- 2011 014 181
- US-A1- 2011 034 860
- US-A1- 2011 034 860
- US-A1- 2016 339 223

## Description

### Technical Field

The present invention relates generally to a microneedle array and, more particularly, to a microneedle array for transcutaneous delivery of a substance such as a drug.

### Background Art

Drug delivery systems (DDSs) refer to a series of techniques for delivering a substance having pharmacological activity to cells, tissues, and organs using various physicochemical techniques.

Drug delivery systems most commonly use an oral administration method, while transcutaneous delivery systems deliver drugs to a portion of the body. Among them, a drug delivery method using a syringe has been widely used for a long time by a method of delivering a liquid drug through a patient's skin with a metal needle.

However, the drug delivery system using a syringe has disadvantages in that it causes inconvenience due to repeated inoculation and pain to the patient whenever the drug is injected, and also causes the patient to be infected due to reuse of the injection needle due to the lack of management of the syringe.

In addition, since the above method requires an inoculator having knowledge of the use of a syringe, there is a disadvantage in that the patient cannot personally administer the drug using a syringe.

Therefore, in recent years, a microneedle array having a plurality of micro-sized percutaneous transmission type microneedles each of which is much smaller than a needle of a pen-type syringe has been made and used for improving a syringe-type drug delivery system.

A microneedle array is a system for percutaneous delivery of a drug through small holes which are physically formed through the stratum corneum. The above system has been actively researched since the beginning of 1998, when the possibility that a silicon microneedle array, which can be manufactured by using the semiconductor process technology, can be applied to drug delivery, is originally proposed from a study by the Georgia Institute of Technology. Currently, these microneedle arrays are made in various sizes and shapes based on various materials such as metals, polymers, glass and ceramics as well as silicon.

In addition, such microneedle arrays are used for delivery of active substances such as drugs and vaccines into a living system, detection and biopsy of analytes in the body, and the injection of other cosmetic substances or drugs into the skin tissue, as well as for the purpose of extracting body fluids such as bloods from subcutis. Accordingly, the microneedle array may be considered one of the drug delivery methods that has been rapidly used in various fields in recent years, since it can perform localized and sustained drug injection, and can minimize pain upon insertion into the skin.

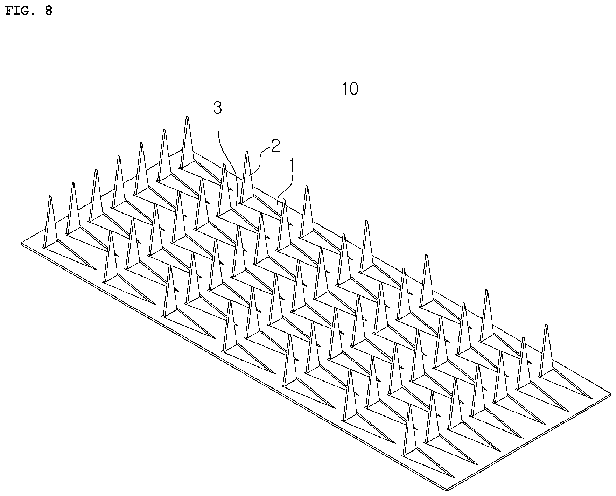

However, the conventional microneedle array has a structural drawback that it does not rapidly spread the drug into the body due to the stratum corneum of the skin. That is, since the conventional microneedle array 10 has a simple structure having a substrate 1 attached to an adhesive sheet (not shown) and a plurality of microneedles 2 protruding from the substrate 1 in an arrangement in which the microneedles 2 are arranged on the substrate 1 in a matrix pattern with a specified interval, as shown in FIG. 8, drug diffusion using transdermal delivery is inefficient.

Accordingly, in recent years, in order to improve a drug delivery rate, transdermal drug delivery has been continuously developed using chemical enhancers, iontophoresis, electroporation, and ultrasound and heat elements. However, this solution not only complicates the manufacturing process, but also increases the manufacturing cost of the microneedle array. In addition, the solution is often unsuitable depending on the form of the drug and may cause side effects to the skin.

Typically, the microneedle array 10 is manufactured in such a way that the substrate 1 is formed by a mold, and the molded substrate is subsequently subjected to pressure in a press. Then, the plurality of microneedles 2 are pressed so as to be bent and protrude from the substrate 1.

However, since the conventional microneedle array 10 has a structure in which the microneedles 2 are arranged on the substrate 1 at a predetermined interval as described above, the press machine should be equipped with a complicated movable die having pressing punches by which the corresponding microneedles 2 are punched and formed.

Further, another problem arises in that, when the plurality of microneedles are pressed by the movable die of the press machine, portions of the substrate 1 disposed between the microneedles 2 are deformed or damaged due to the influence of the pressing punches.

In particular in the case that the number of the microneedles 2 are increased so that the distance between the microneedles 2 becomes narrowed so as to enhance a transdermal drug delivery rate, the portions of the substrate 1 disposed between the microneedles 2 are disposed closer to the pressing punches and may be easily deformed or broken.

Accordingly, the applicant has proposed a novel microneedle array of the present invention in order to solve the above problems, and related prior art documents include Unexamined Korean Patent Publication No. 10-2014-0105686 entitled 'Microneedle patch for stimulating the body's painful parts or acupuncture points', as well as US 2016/339223 A1, US 2007/293815 A1, US 2007/177858 A1, US 2007/161964 A1, US 2011/034860 A1, US2011(014181 A1 and US 2007/049901 A1, which all relate to a substrate with a microneedle array.

Further, since the conventional microneedle array requires separate injection devices or prolonged contact with the user's skin in order to smoothly deliver the drug into the body through the shorter microneedles, the conventional microneedle array also has problems in that it is troublesome to use and side effects such as inflammation due to the material of the microneedles may occur.

To solve these problems, the applicant has proposed a microneedle array made of biodegradable metals through patent applications and the like. The proposed microneedle array has excellent drug delivery capability relative to a conventional microneedle array since the microneedles are formed from a metal sheet material having high permeability to the stratum corneum. However, in order to allow a drug to be injected from the outside of the skin using a drug-carrying patch or the like without using a separate drug injection device or the like, the proposed microneedle array requires a faster and more effective injection structure.

### Disclosure

### Technical Problem

Accordingly, the present invention has been made keeping in mind the above problems occurring in the related art, and an object of the present invention is to provide a microneedle array having a multi-type microneedle units arranged to allow a drug to be rapidly delivered to a transdermal tissue of the body and to achieve convenience in manufacturing the microneedle array.

Another object of the present invention is to provide a biodegradable metal plate-type microneedle array capable of injecting a drug more quickly and smoothly without using a separate injection mechanism or the like.

### Technical Solution

In accordance with the present invention, a microneedle array for subcutaneous insertion having the features of claim 1 is proposed, which includes: a substrate; a plurality of needle openings provided in the substrate; and a plurality of needle units disposed around respective needle openings and each having a plurality of microneedles circumferentially arranged at regular intervals around a respective needle opening to protrude from the substrate so as to be inserted into the skin, wherein the substrate and the microneedles are formed of a bioabsorbable metal, wherein the bioabsorbable metal includes at least one element of magnesium and zinc generating hydrogen gas and MgCl, or hydrogen gas and ZnO in a reaction with water, in a decomposition process when inserted into the skin, wherein the microneedle includes a first needle section having a first end connected to the substrate, and a second needle section having a first end connected to a second end of the first needle section, with the first and second needle sections protruding in vertical directions from a surface of the substrate, wherein the first needle section has a gradually narrower width from the first end to the second end thereof in a longitudinal direction, wherein the microneedle includes a carrying recess having a first end partially disposed in the substrate and a second end disposed to extend toward a distal end of the microneedle in a longitudinal direction thereof and a carrying hole being provided in the carrying recess, and wherein a cutout portion is provided at a connection between the first needle section and the substrate, the cutout portion being formed by cutout slits respectively disposed between the opposite widthwise ends of the first needle section and the first end of the carrying hole at the first end of the first needle section.

The needle opening may be provided in the substrate in a circular or regular polygonal shape.

The microneedles of the respective needle unit may be arranged such that, when the needle opening has a regular polygonal shape, the microneedles are respectively provided on the substrate at middle portions of respective sides of the needle opening.

The second needle section may be provided in the form of an arrowhead having a gradually narrower width from the first end to the second end thereof in the longitudinal direction, and may have, on the first end thereof, an engagement protrusion having a width greater than that of the second end of the first needle section.

The carrying recess may be provided in the microneedle or the substrate to provide a flow path for a drug.

A carrying slit may be further provided to communicatingly extend from the second end of the carrying recess to the distal end of the microneedle.

The carrying hole may have an inner diameter gradually decreasing from top toward bottom of the first or second needle section.

The substrate may have a honeycomb structure when the needle opening is provided in the form of a regular hexagon.

The bioabsorbable metal may contain at least one element of magnesium, calcium, zinc, and iron.

### Advantageous Effects

According to the present invention having the above-described characteristics, the multi-type microneedle array has a structure in which the plurality of microneedles are arranged in a predetermined pattern around one needle opening, so that the microneedles can be inserted into a predetermined area of skin so as to rapidly deliver a drug to a transdermal tissue.

Since the multi-type microneedle array can have a honeycomb structure by needle openings formed in the form of a regular hexagon, in the process of pressing and bending the microneedles, the substrate can be restricted from being deformed or damaged.

Since the multi-type microneedle array can be provided such that the plurality of microneedles can protrude from the substrate without using the same number of punches provided on the movable die of the press machine as the number of the microneedles, thereby reducing the manufacturing cost of the movable die of the press machine and therefore manufacturing the microneedle array in an economical and simple manner.

The multi-type microneedle array may transmit a drug for treatment into the body, as well as components (magnesium, calcium, zinc, iron, etc.) contained in a bioabsorbable metal so as to supply minerals into the body along with the drug.

In the microneedle array using the bioabsorbable metal, since the microneedles may be easily left under the skin with a simple process of separating the substrate contacting the skin from the skin, a user can receive the bioabsorbable metal ions, which are beneficial to the human body, in the body without being subjected to any other procedure.

Since the microneedle array has a structure in the microneedle so as to facilitate the flow of an active ingredient such as a drug, thereby allowing the active ingredient to more quickly and smoothly pass and spread into the skin through the stratum corneum.

### Description of Drawings

FIG. 1 is a plan view illustrating a multi-type microneedle array according to the present invention;
FIG. 2 is an enlarged view of Section A shown in FIG. 1;
FIG. 3 is a perspective view illustrating a state in which microneedles are erected according to the present invention;
FIG. 4 is a cross-sectional view illustrating the microneedle having a carrying recess and a carrying hole according to the present invention;
FIG. 5 shows plan views illustrating the microneedle having a cutout portion according to present invention;
FIG. 6 is a plan view illustrating a circular needle opening according to an embodiment of the present invention;
FIG. 7 is a plan view illustrating a regular octagonal needle opening according to another embodiment of the present invention; and
FIG. 8 is a perspective view illustrating a conventional microneedle array.

### Mode for Invention

The advantages and features of the present invention and the method of achieving them will become apparent with reference to the below together with the accompanying drawings.

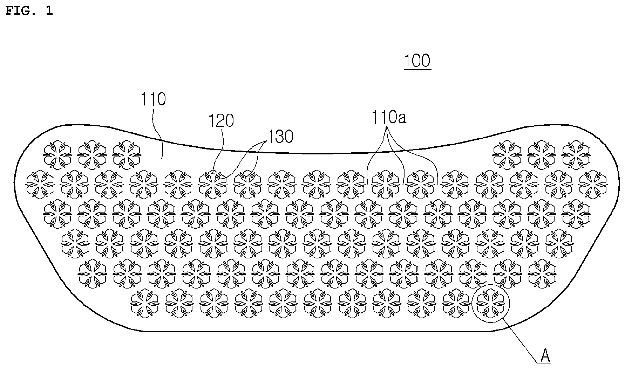

Hereinafter, a microneedle array according to the present invention will be described in detail with reference to FIGS. 1 to 7. In the following description, a detailed description of known functions and configurations incorporated herein will be omitted so as to avoid obscuring the subject matter of the present invention.

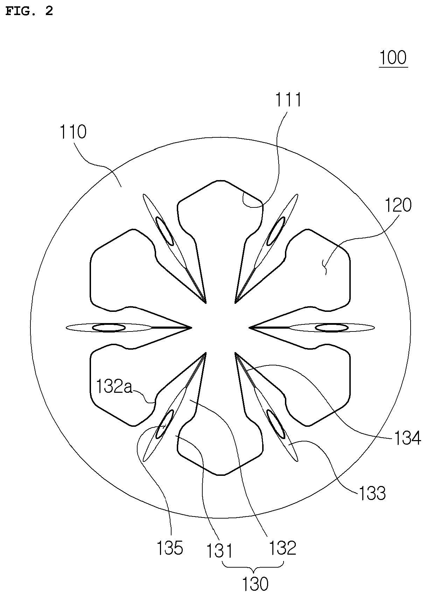

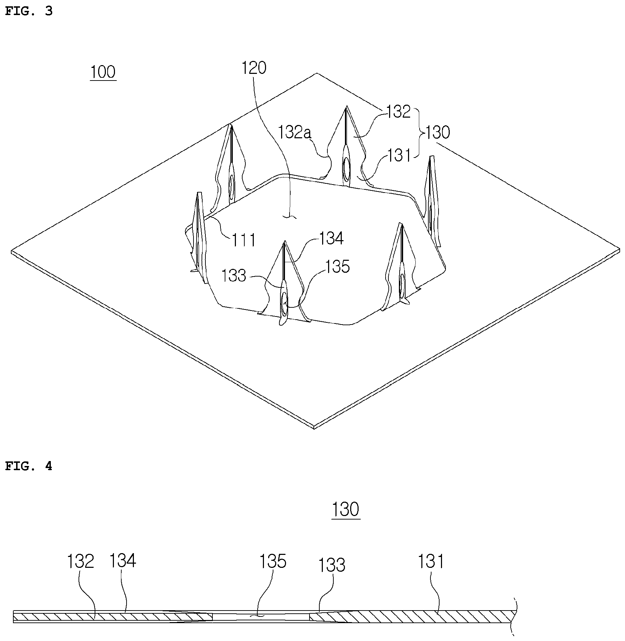

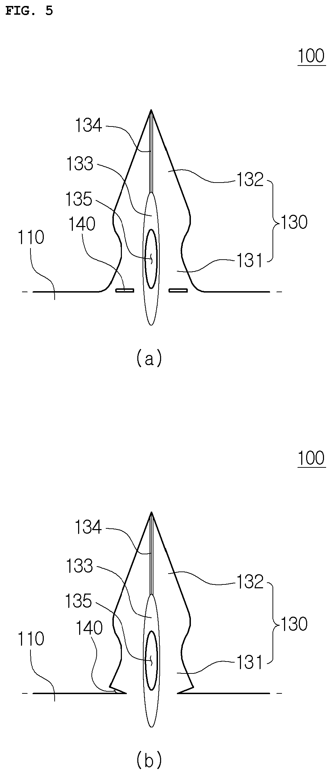

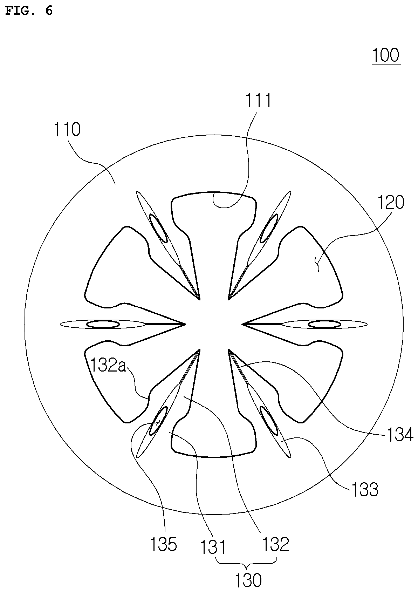

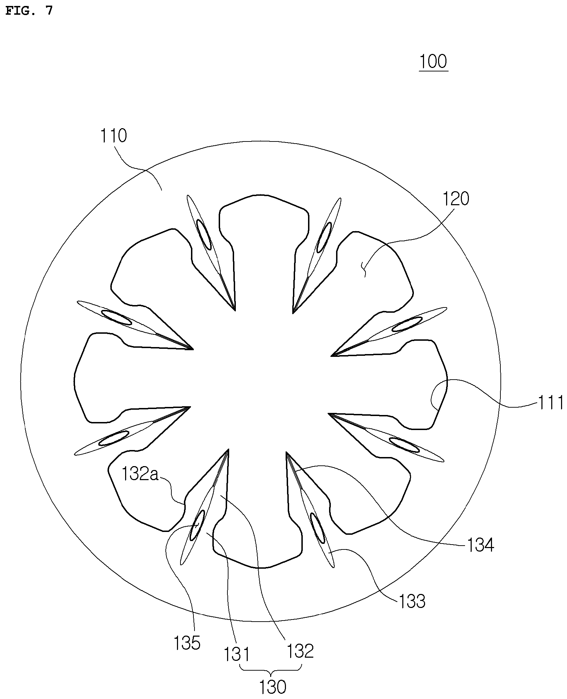

FIG. 1 is a plan view illustrating a multi-type microneedle array according to the present invention; FIG. 2 is an enlarged view of Section A shown in FIG. 1; FIG. 3 is a perspective view illustrating a state in which microneedles are erected according to the present invention; FIG. 4 is a cross-sectional view illustrating the microneedle having a carrying recess and a carrying hole according to the present invention; FIG. 5 shows plan views illustrating the microneedle having a cutout portion according to the present invention; FIG. 6 is a plan view illustrating a circular needle opening according to an embodiment of the present invention; FIG. 7 is a plan view illustrating a regular octagonal needle opening according to another embodiment of the present invention; and FIG. 8 is a perspective view illustrating a conventional microneedle array.

As illustrated in FIGS. 1 to 7, the microneedle array 100 includes a substrate 110 having a plurality of needle openings 120, and a plurality of needle units disposed around respective needle openings and each having a plurality of microneedles 130 circumferentially arranged at regular intervals around respective needle opening to protrude from the substrate 110.

The substrate 110 may be in the form of a thin sheet having a predetermined surface area and thickness, and may be attached to the user's skin in the form of a patch while being placed on an adhesive base sheet (not shown) applied with an adhesive material

In addition, the substrate 110 may be provided in various sizes and shapes corresponding to a target skin region, and the periphery of the substrate 110 may be formed to have various curvatures so as to be in tight contact with the curved skin region. For example, when the substrate 110 is attached to the user's nose, the substrate 110 may be formed in the same shape as a known nose pack.

In addition, the substrate 110 may carry a drug intended to be delivered to a subcutaneous tissue. The substrate 110 may carry the drug by various known methods such as the coating of the drug accomplished through immersion of the substrate 110 into a container storing the drug therein, the coating of the drug accomplished through application of the drug onto the substrate 110, and the like.

For example, the drug to be carried on the substrate 110 may be not only a drug for prevention and treatment of diseases, but also a genetic material or Epidermal Growth Factor (EGF) or Hyaluronic acid for skin care.

The needle openings 120 are components formed by processing the substrate 110 with a laser cutting device. As described above, the needle openings 120 are formed at regular intervals on the surface of the substrate 110.

The needle opening 120 may be provided in the substrate 110 in the form of a circular or a regular polygonal shape. For example, the needle opening may be formed in the substrate 110 in the form of a regular hexagonal or regular octagonal shape as illustrated in FIGS. 2 and 7, or the circular shape as illustrated in FIG. 6.

As illustrated in FIG. 2, the microneedle 130 includes a first needle section 131 having a first end connected to the substrate 110 and a second end opposite to the first end, and a second needle section 132 having a first end connected to the second end of the first needle section 131 and a second end formed as a distal end of the microneedle opposite to the first end of the second needle section, wherein the first and second needle sections are provided in the needle opening 120.

The microneedle 130 configured as described above may be bent and protruded in a vertical direction from the surface of the substrate 110 by a molding process or a pressing process using a press machine. That is, the microneedle 130 may be a portion that is inserted under the user's skin to deliver the drug when the substrate 110 contacts the user's skin.

The microneedles 130 of the respective needle unit may be arranged such that, when the needle opening 120 has the regular polygonal shape, the microneedles are respectively provided on the substrate at middle portions of respective sides 111 of the needle opening 120.

If the microneedles 130 of the needle unit are provided on the substrate 110 at respective corners of the needle opening 120, the microneedles 130 may be broken in the process of bending the microneedles 130 in a vertical direction (in the pressing process by a pressing machine). Therefore, in order to prevent the microneedles 130 from being broken, the microneedles 130 are preferably provided on the substrate 110 at middle portions of respective sides 111 of the needle opening 120.

The first and second needle sections 131 and 132 of the microneedle 130 may have a shape of an arrowhead as a whole so that the needle sections can be easily inserted into the skin. The first needle section 131 may be provided in the form of an arrowhead having a gradually narrower width from the first end to the second end thereof in the longitudinal direction.

The second needle section 132 may also be provided in the form of an arrowhead having a gradually narrower width from the first end to the second end thereof in the longitudinal direction, and may have, on the first end thereof, an engagement protrusion 132a having a width greater than that of the second end of the first needle section.

A carrying recess 133 is also provided in the microneedle 130 or the substrate 110 to provide a flow path for a drug.

The carrying recess 133 is provided in one side or the other side of the substrate 110, and has a first end partially disposed in the substrate 110 and a second end disposed to extend toward the distal end of the microneedle 130, i.e. the second end of the second needle section 132, in a longitudinal direction thereof.

The carrying recess 133 may be selectively provided in one side or the other side of the substrate 110, or otherwise in both sides of the substrate 110.

The carrying recess 133 serves to form a flow path for a drug carried in the substrate 110 or the microneedle 130 and to provide a space for a drug carried in the substrate 110 or the microneedle 130 so as to adjust the amount of drug delivered into the body.

As illustrated in FIG. 4, the carrying recess 133 may have a shape in which the inner diameter gradually decreases in the thickness direction of the substrate 110 or the microneedle 130.

The carrying recess 133 may have a shape to increase the area of the substrate 110 or the microneedle 130 so as to allow the amount of the drug carried in the substrate 110 or the microneedle 130 while providing a flow path for the drug carried in the substrate 110 or the microneedle 130.

In other words, if the microneedle 130 is made of a flat surface without the carrying recess 133 as a drug delivery path, the microneedle 130 has a structure in which when inserted into the skin, it is in close contact with the skin so that the drug may not easily flow therethrough. On the contrary, when the carrying recess 133 is formed in the substrate 110 and the microneedle 130, the microneedle may have a structure in which when inserted into the skin, it allows easy delivery of the drug stored in the carrying recess 133 into the body. In addition, the drug carried on the substrate 110 or the microneedle 130 may flow along the forming direction of the carrying recess 133 and be delivered subcutaneously.

A carrying slit 134 may be further provided to the microneedle to communicatingly extend from the second end of the carrying recess 133 to the distal end of the microneedle 130, i.e. to the second end of the second needle section 132.

The carrying slit 134 functions to allow the drug stored in the carrying recess 133 or a carrying hole 135 to be described later to easily flow to the tip of the microneedle 130 so that the drug contained in the substrate 110 or the microneedle 130 can be more easily delivered to the user's subcutaneous tissue sequentially through the carrying recess 133 and the carrying slit 134.

The carrying hole 135 is provided in the carrying recess 133. That is, the carrying hole 135 is provided in the portion of the substrate 110 or the microneedle 130 where the carrying recess 133 is formed, along a forming direction of the carrying recess 133. That is, the carrying hole 135 may be provided in the carrying recess 133 along the longitudinal direction of the first needle section 131 or the second needle section 132 of the microneedle 130.

Since the carrying hole 135 communicatingly connects both sides of the substrate 110 or the microneedle 130, the carrying hole 135 allows the drug contained in one side or the other side of the substrate 110 or the microneedle 130 to communicate with each other, thereby enabling rapid diffusion of the drug into the user's skin.

In addition, since the carrying hole 135 provides a space for storing a drug as in the carrying recess 133, the amount of the drug to be carried on the substrate 110 or the microneedle 130 can be regulated. That is, since the carrying hole 135 can be formed in such a manner that the inner diameter gradually decreases from one side toward the other side and vice versa of the first needle section 131 or the second needle section 132, a space can be provided in which a drug coating layer formed through immersion is accommodated in the microneedle 130.

The provision of the above-mentioned carrying recess 133, the carrying slit 134, and the carrying hole, which form a drug flow path in the surface of the microneedle 130 and the contact surface of the microneedle with the skin (e.g. the stratum corneum) may be easily adapted to the microneedle array 100, as well as the microneedle array of FIG. 8 having a structure in which one microneedle is provided in one needle opening.

Since the microneedle array 100 according to the present invention is constructed so that the microneedles 130 are provided at regular intervals on the substrate 110 around the needle opening 120, drugs intended to be supplied into the body can be intensively delivered to the subcutaneous tissues and rapidly spread.

In addition, when the needle openings 120 are formed in the shape of a regular hexagon, the substrate 110 may have a honeycomb structure. With the honeycomb structure of the substrate 110, the portions (110a, FIG. 1) of the substrate 110 defining the needle openings 120 may be prevented from being deformed or broken in the process of pressing and bending the microneedles 130 disposed around the needle openings 120 with punches 110 of a press machine.

More specifically, the microneedles 130 are pressed and bent when the punches of the movable die of a press machine are inserted into the needle openings 120. Here, there is a possibility that the portions 110a (see FIG. 1) of the substrate 110 disposed on the substrate between the needle openings 120 may be deformed or torn due to the influence of the punches of the press machine.

However, the microneedle array 100 according to the present invention may form the needle openings 120 in a regular hexagon so that the substrate 110 may be formed in a honeycomb structure. This increases the whole strength of the substrate 110 so that the substrate 110 can be prevented from being deformed or broken during a work process using a press machine.

In addition, the plurality of microneedles 130 may be pressed and bent by a single pressing punch to be inserted into one needle opening 120. Thus, there is no need to provide the same number of the pressing punches provided on the movable die of the press machine as the number of the microneedles 130, thereby simplifying the whole structure of the movable die of the press machine. Thus, the manufacturing cost of the movable die mat be reduced and the manufacturing cost and process of the multi-type microneedle array 100 may be reduced and simplified.

Meanwhile, the microneedle array 100 according to the present invention is formed of bioabsorbable metal. That is, the substrate 110 or the microneedle 130 are formed of bioabsorbable metal composed of components beneficial to the human body.

That is, the substrate 110 may be made of metal as a bioabsorbable metal including at least one component of magnesium, calcium, zinc, and iron, so that the microneedles 130 provided on the substrate 110 may also be made of a bioabsorbable metal.

In addition, when the substrate 110 is formed of a bioabsorbable metal, a cutout portion 140 is further formed at a connection between the first needle section 131 and the substrate 110.

When the cutout portion 140 is torn upon separation of the bioabsorbable substrate 110 from the user's skin, the microneedles 130 may be separated from the substrate 110 and remain on the skin.

Here, as illustrated in FIGS. 5(a) and 5(b), the cutout portion 140 are provided on opposite widthwise sides of the first end of the first needle section 131.

That is, the cutout portion is cutout slits respectively disposed between the opposite widthwise ends of the first needle section 131 and the first end of the carrying recess 133 at the first end of the first needle section 131 as illustrated in FIG. 5(a), while the cutout portion 140 may have notches disposed at the opposite widthwise ends of the first end of the first needle section 131 so as to communicate with the needle opening 120.

The cutout portion 140 is configured to allow the first needle section 131 and the second needle section 132, which are inserted subcutaneously, to be subcutaneously left when the substrate 110 contacting the skin is separated from the skin, and serves to reduce an area of a connection between the first needle section 131 and the substrate 110 so that the first end of the first needle section 131 is easily broken from the substrate 110.

That is, the first needle section 131 and the second needle section 132 inserted under the skin can be stuck to the subcutaneous tissue by the contractive force of the subcutaneous tissue. Here, when the first needle section 131 and the second needle section 132 are pulled by the separation force of the substrate 110 from the skin, which is larger than the contraction force of the subcutaneous tissue, the first needle section 131 and the second needle section 132 are forced to separate from the skin together with the substrate 110.

However, the cutout portion 140 is formed at the connection between the substrate 110 and the first needle section 131, so that the force exerted to the substrate 110 to pull the first needle section 122 and the second needle section 123 is reduced so as to be smaller than the contraction force of the subcutaneous tissue, thereby allowing the first needle section 131 and the second needle section 132 to be left under the skin.

The first needle section 131 and the second needle section 132 can be further adhered to the subcutaneous tissue since the engagement protrusion 132a formed on the second needle section 132 can be caught in the subcutaneous tissue. Therefore, when the substrate 110, which is in contact with the skin, is separated from the skin, the connection between the first needle section 131 and the substrate 110 can be more easily broken by the engagement protrusion 132a.

The first needle section 131 and the second needle section 132 remaining on the skin can subcutaneously deliver the drug carried in the substrate 110 as well as minerals contained in the bioabsorbable metal. That is, magnesium, calcium, zinc, and iron components used as bioabsorbable metals can be subcutaneously transmitted.

For reference, bioabsorbable metals have been made commercially for the application of magnesium based alloy for orthopedic implants. Bioabsorbable metals applied to orthopedic implants were focused on reducing a decomposing rate thereof and improving the corrosion resistance thereof for safe fixation of the fracture.

However, unlike the bioabsorbable metals used for orthopedic surgery, the bioabsorbable metals forming the microneedle array 100 according to the present invention accelerates the decomposition rate in the body, so that mechanisms may be applied to supply minerals along with drug release under the skin.

For example, magnesium, calcium, and zinc used as bioabsorbable metals have a mechanism of reacting with water to produce hydrogen gas, as shown in the following Chemical Formulas 1 to 3, respectively.

Mg + 2H₂O → Mg(OH)₂ + H₂ (gas)

Ca + 2H₂O → Ca(OH)₂ + H₂ (gas)

Zn + 2H₂O → Zn(OH)₂ + H₂ (gas)

The substrate 110 and the microneedles 130 formed of the bioabsorbable metal as described above may emit ions and decomposition products under the skin and the hydrogen gas generated by the byproducts provides a swelling effect in the subcutaneous space, thereby being effective against wrinkles.

In addition, ZnO and MgCl, which are byproducts formed by magnesium and zinc, which are inserted into the body as components of the bioabsorbable metal, remain under the skin and serve as a drug delivery enhancer improving the subcutaneous delivery of a drug carried in the substrate 110 and the microneedles 130. Therefore, the substrate 110 and the microneedle 130, which are formed of the bioabsorbable metal, can effectively deliver the carried drug to the user.

The shapes of the needle openings 120 and the microneedles 130 formed on the substrate 110 may be patterned on the substrate 110 by a known lithography or etching technique.

At this time, since the substrate 110 formed of a bioabsorbable metal has lower corrosion resistance than a metal material such as stainless steel or iron, the periphery of the microneedle 130 becomes corroded and thinner than the thickness of the original material itself so as to be sharpened by the lithography or etching technique. That is, at the periphery of the first needle section 131 or the second needle section 132, a sharp inclined surface having a thickness smaller than the thickness of the first needle 131 or the second needle 132 is formed by the lithography or etching technique, so that the sharp inclined surface can be easily inserted subcutaneously.

Also, since the multi-type microneedle array 100 according to the present invention has the needle opening 120 having a relatively large diameter or area rather than the needle opening formed in the conventional microneedle array, a light therapy can also be performed through the needle opening 120 in parallel.

In other words, even if the substrate 110 is attached to the skin, the user's skin can be exposed to the outside through the plurality of needle openings 120, so that the exposed skin can be easily exposed to light beneficial to treatment for skin diseases such as skin pain, acne, atopy, thereby perform the light treatment.

Therefore, since a user can be treated by the drug injected into the skin through the microneedles 130 and by the light irradiated through the needle openings 120, a synergy effect between the drug treatment and the light therapy can be expected.

For example, although described in the course of the present invention, beneficial light exposed to the skin through the needle openings 120 may be irradiated to the body to receive light therapy, the present invention is not limited thereto. That is, a skin care substance or a skin treatment substance in a liquid state can be applied to the skin exposed through the needle openings 120 to perform a skin treatment.

In addition, the substrate 110 can be brought into close contact with the user's skin through the needle openings 120.

That is, since the needle opening 120 of the present invention accommodates the plurality of microneedles 130 in one space so that the needle opening has a relatively large diameter or area than that of the needle opening (which can accommodate only one microneedle) of the conventional microneedle array, when the substrate 110 is in contact with the user's skin, the user's skin is easily accommodated by the needle opening 120. Here, a negative pressure is formed at the same time, so that the substrate 110 can be more closely adhered to the skin.

Therefore, the microneedle array 100 according to the present invention may have a configuration in which the substrate 110 can be more closely attached to the user's skin by the needle openings 120.

Since the multi-type microneedle array 100 according to the present invention has a structure in which the plurality of microneedles are arranged in a predetermined pattern around a single needle opening, the plurality of microneedles can be inserted into the skin of a predetermined area, so that a drug can be rapidly delivered into the body.

Since the multi-type microneedle array 100 according to the present invention has a structure in which the substrate 110 has a honeycomb structure by provision of regular hexagonal needle openings 120, the substrate 110 may be prevented from being deformed or broken in the process of pressing and bending the microneedles 130.

In the multi-type microneedle array 100 according to the present invention, the plurality of microneedles 130 can protrude from the substrate 110 without having the number of the pressing punches provided on the movable mold of the press machine be equal to the number of the microneedles 130, thereby reducing the manufacturing cost of the movable mold of the press, and thus obtaining reduced manufacturing cost of the microneedles and the simplified manufacturing process.

### Industrial Applicability

The microneedle array according to the present invention may be applicable to a variety of industrial fields such as medical field, skin beauty field, and the like.

## Claims

1. A microneedle array (100) for subcutaneous insertion, comprising:
a substrate (110);
a plurality of needle openings (120) provided in the substrate (110); and
a plurality of needle units disposed around respective needle openings (120) and each having a plurality of microneedles (130) circumferentially arranged at regular intervals around a respective needle opening (120) to protrude from the substrate (110) so as to be inserted into the skin,
wherein the substrate (110) and the microneedles (130) are formed of a bioabsorbable metal, wherein the bioabsorbable metal includes at least one element of magnesium and zinc generating hydrogen gas and MgCl, or hydrogen gas and ZnO in a reaction with water, in a decomposition process when inserted into the skin, wherein the microneedle (130) includes a first needle section (131) having a first end connected to the substrate (110), and a second needle section (132) having a first end connected to a second end of the first needle section (131), with the first and second needle sections protruding in vertical directions from a surface of the substrate (110),
wherein the first needle section (131) has a gradually narrower width from the first end to the second end thereof in a longitudinal direction,
**characterized in that**
the microneedle (130) includes a carrying recess (133) having a first end partially disposed in the substrate (110) and a second end disposed to extend toward a distal end of the microneedle (130) in a longitudinal direction thereof and a carrying hole (135) being provided in the carrying recess (133),
wherein a cutout portion (140) is provided at a connection between the first needle section (131) and the substrate (110), the cutout portion (140) being formed by cutout slits respectively disposed between the opposite widthwise ends of the first needle section (131) and the first end of the carrying hole (135) at the first end of the first needle section (131).

2. The microneedle array (100) of claim 1, wherein the needle opening (120) is provided in the substrate (110) in a circular or regular polygonal shape.

3. The microneedle array (100) of claim 2, wherein the microneedles (130) of the respective needle unit are arranged such that, when the needle opening (120) has the regular polygonal shape, the microneedles (130) are respectively provided on the substrate (110) at middle portions of respective sides (111) of the needle opening (120).

4. The microneedle array (100) of claim 1, wherein the second needle section (132) is provided in the form of an arrowhead having a gradually narrower width from the first end to the second end thereof in the longitudinal direction, and has, on the first end thereof, an engagement protrusion having a width greater than that of the second end of the first needle section (131).

5. The microneedle array (100) of claim 4, wherein the carrying recess (133) is provided in the microneedle (130) to provide a flow path for a drug.

6. The microneedle array (100) of claim 5, wherein a carrying slit (134) is further provided to communicatingly extend from the second end of the carrying recess (133) to the distal end of the microneedle (130).

7. The microneedle array (100) of claim 5, the carrying hole (135) is configured to have an inner diameter gradually decreasing from top toward bottom of the first or second needle section (131, 132).

8. The microneedle array (100) of claim 2, wherein the substrate (110) has a honeycomb structure when the needle opening (120) is provided in the form of a regular hexagon.

9. The microneedle array (100) of claim 1, wherein the bioabsorbable metal further contains at least one element of calcium and iron.

## Patentansprüche

1. Mikronadelanordnung (100) zum subkutanen Einführen, umfassend:
ein Substrat (110);
eine Vielzahl von Nadelöffnungen (120), die in dem Substrat (110) vorgesehen sind; und
eine Vielzahl von Nadeleinheiten, die um jeweilige Nadelöffnungen (120) herum angeordnet sind und jeweils eine Vielzahl von Mikronadeln (130) umfassen, die in regelmäßigen Abständen um eine jeweilige Nadelöffnung (120) herum angeordnet sind, um aus dem Substrat (110) herauszuragen, so dass sie in die Haut eingeführt werden können,
wobei das Substrat (110) und die Mikronadeln (130) aus einem bioabsorbierbaren Metall gebildet sind, wobei das bioabsorbierbare Metall mindestens ein Element von Magnesium und Zink enthält, welches Wasserstoffgas und MgCl oder Wasserstoffgas und ZnO in einer Reaktion mit Wasser in einem Zersetzungsprozess erzeugt, wenn es in die Haut eingeführt wird, wobei die Mikronadel (130) einen ersten Nadelabschnitt (131) mit einem ersten Ende, welches mit dem Substrat (110) verbunden ist, und einen zweiten Nadelabschnitt (132) mit einem ersten Ende, welches mit einem zweiten Ende des ersten Nadelabschnitts (131) verbunden ist, umfasst, wobei der erste und der zweite Nadelabschnitt in vertikalen Richtungen von einer Oberfläche des Substrats (110) vorstehen,
wobei der erste Nadelabschnitt (131) eine allmählich schmaler werdende Breite von seinem ersten Ende zu seinem zweiten Ende in einer Längsrichtung hat,
**dadurch gekennzeichnet, dass**
die Mikronadel (130) einen Trageausschnitt (133) mit einem ersten Ende, welches teilweise in dem Substrat (110) angeordnet ist, und einem zweiten Ende, welches so angeordnet ist, dass es sich in Richtung eines distalen Endes der Mikronadel (130) in einer Längsrichtung davon erstreckt, und ein Trageloch (135), das in dem Trageausschnitt (133) vorgesehen ist, umfasst,
wobei ein ausgeschnittener Teil (140) an einer Verbindung zwischen dem ersten Nadelabschnitt (131) und dem Substrat (110) vorgesehen ist, wobei der ausgeschnittene Abschnitt (140) durch ausgeschnittene Schlitze gebildet wird, welche jeweils zwischen den der Breite nach gegenüberliegenden Enden des ersten Nadelabschnitts (131) und dem ersten Ende des Tragelochs (135) am ersten Ende des ersten Nadelabschnitts (131) angeordnet sind.

2. Mikronadelanordnung (100) nach Anspruch 1, wobei die Nadelöffnung (120) in dem Substrat (110) in einer kreisförmigen oder regelmäßig polygonalen Form vorgesehen ist.

3. Die Mikronadelanordnung (100) nach Anspruch 2, wobei die Mikronadeln (130) der jeweiligen Nadeleinheit so angeordnet sind, dass, wenn die Nadelöffnung (120) die regelmäßige polygonale Form hat, die Mikronadeln (130) jeweils auf dem Substrat (110) an mittleren Teilen der jeweiligen Seiten (111) der Nadelöffnung (120) vorgesehen sind.

4. Mikronadelanordnung (100) nach Anspruch 1, wobei der zweite Nadelabschnitt (132) in Form einer Pfeilspitze vorgesehen ist, welche in Längsrichtung von ihrem ersten Ende zu ihrem zweiten Ende hin eine allmählich schmalere Breite hat, und an ihrem ersten Ende einen Eingriffsvorsprung hat, dessen Breite größer ist als die des zweiten Endes des ersten Nadelabschnitts (131).

5. Mikronadelanordnung (100) nach Anspruch 4, wobei der Trageausschnitt (133) in der Mikronadel (130) vorgesehen ist, um einen Strömungsweg für ein Arzneimittel zu schaffen.

6. Mikronadelanordnung (100) nach Anspruch 5, wobei ferner ein Trageschlitz (134) vorgesehen ist, um sich in Verbindung stehend vom zweiten Ende des Trageausschnitts (133) zum distalen Ende der Mikronadel (130) zu erstrecken.

7. Mikronadelanordnung (100) nach Anspruch 5, wobei das Trageloch (135) so konfiguriert ist, dass es einen Innendurchmesser umfasst, der vom oberen Ende zum unteren Ende des ersten oder zweiten Nadelabschnitts (131, 132) allmählich abnimmt.

8. Mikronadelanordnung (100) nach Anspruch 2, wobei das Substrat (110) eine Wabenstruktur umfasst, wenn die Nadel Öffnung (120) in Form eines regelmäßigen Sechsecks vorgesehen ist.

9. Mikronadelanordnung (100) nach Anspruch 1, wobei das bioabsorbierbare Metall außerdem mindestens ein Element von Kalzium und Eisen enthält.

## Revendications

1. Réseau de micro-aiguilles (100) pour insertion sous-cutanée, comprenant :
un substrat (110) ;
une pluralité d'ouvertures d'aiguille (120) prévues dans le substrat (110) ; et
une pluralité d'unités d'aiguille disposées autour d'ouvertures d'aiguille respectives (120) et
chacune ayant une pluralité de micro-aiguilles (130) disposées circonférentiellement à intervalles réguliers autour d'une ouverture d'aiguille respective (120) pour faire saillie du substrat (110) afin d'être insérée dans la peau,
dans lequel le substrat (110) et les micro-aiguilles (130) sont formés d'un métal bioabsorbable, dans lequel le métal bioabsorbable comprend au moins un élément de magnésium et de zinc générant de l'hydrogène gazeux et du MgCl, ou de l'hydrogène gazeux et du ZnO dans une réaction avec de l'eau, dans un processus de décomposition lorsqu'il est inséré dans la peau, dans lequel la micro-aiguille (130) comprend une première section d'aiguille (131) dont la première extrémité est reliée au substrat (110), et une deuxième section d'aiguille (132) dont la première extrémité est reliée à la deuxième extrémité de la première section d'aiguille (131), la première et la deuxième sections d'aiguille faisant saillie verticalement à partir d'une surface du substrat (110), dans laquelle la première section d'aiguille (131) présente une largeur progressivement plus étroite de sa première à sa deuxième extrémité dans le sens longitudinal,
**caractérisé en ce que**
la micro-aiguille (130) comprend une cavité de transport (133) ayant une première extrémité partiellement disposée dans le substrat (110) et une seconde extrémité disposée pour s'étendre vers une extrémité distale de la micro-aiguille (130) dans une direction longitudinale de celle-ci et un trou de transport (135) étant fourni dans la cavité de transport (133),
dans lequel une partie découpée (140) est prévue à une connexion entre la première section d'aiguille (131) et le substrat (110), la partie découpée (140) étant formée par des fentes découpées respectivement disposées entre les extrémités opposées dans le sens de la largeur de la première section d'aiguille (131) et la première extrémité du trou de transport (135) à la première extrémité de la première section d'aiguille (131).

2. Le réseau de micro-aiguilles (100) de la revendication 1, dans lequel l'ouverture de l'aiguille (120) est prévue dans le substrat (110) sous une forme circulaire ou polygonale régulière.

3. Le réseau de micro-aiguilles (100) de la revendication 2, dans lequel les micro-aiguilles (130) de l'unité d'aiguille respective sont disposées de telle sorte que, lorsque l'ouverture de l'aiguille (120) a une forme polygonale régulière, les micro-aiguilles (130) sont respectivement fournies sur le substrat (110) à des parties centrales des côtés respectifs (111) de l'ouverture de l'aiguille (120).

4. Le réseau de micro-aiguilles (100) de la revendication 1, dans lequel la deuxième section d'aiguille (132) la forme d'une pointe de flèche dont la largeur diminue progressivement de la première à la deuxième extrémité dans la direction longitudinale, et comporte, à sa première extrémité, une saillie d'engagement dont la largeur est supérieure à celle de la deuxième extrémité de la première section d'aiguille (131).

5. Le réseau de micro-aiguilles (100) de la revendication 4, dans lequel la cavité de transport (133) est prévue dans la micro-aiguille (130) pour fournir une voie d'écoulement pour un médicament.

6. Le réseau de micro-aiguilles (100) de la revendication 5, dans lequel une fente de transport (134) est en outre prévue pour s'étendre en communication de la seconde extrémité de la cavité de transport (133) à l'extrémité distale de la micro-aiguille (130).

7. Le réseau de micro-aiguilles (100) de la revendication 5, le trou de transport (135) est configuré pour avoir un diamètre intérieur diminuant progressivement du haut vers le bas de la première ou de la deuxième section d'aiguille (131, 132).

8. Le réseau de micro-aiguilles (100) de la revendication 2, dans lequel le substrat (110) a une structure en nid d'abeille lorsque l'ouverture de l'aiguille (120) a la forme d'un hexagone régulier.

9. Le réseau de micro-aiguilles (100) de la revendication 1, dans lequel le métal bioabsorbable contient en outre au moins un élément de calcium et de fer.
